(19) Europäisches Patentamt
European Patent Office
Office européen des brevets

(11) Veröffentlichungsnummer: **0 006 608**
**B1**

(12) **EUROPÄISCHE PATENTSCHRIFT**

(45) Veröffentlichungstag der Patentschrift: **30.06.82**

(51) Int. Cl.³: **C 07 D 213/64, A 01 N 43/40**

(21) Anmeldenummer: **79102118.1**

(22) Anmeldetag: **26.06.79**

(54) **Herbizid wirksame, optisch aktive R(+)-Dichlorpyridyloxy-alpha-phenoxy-propionsäure-propargylester, Verfahren zu ihrer Herstellung und deren Verwendung in Mitteln zur Unkrautbekämpfung.**

(30) Priorität: **29.06.78 CH 7102/78**

(43) Veröffentlichungstag der Anmeldung: **09.01.80 Patentblatt 80/1**

(45) Bekanntmachung des Hinweises auf die Patenterteilung: **30.06.82 Patentblatt 82/26**

(84) Benannte Vertragsstaaten: **AT BE CH DE FR GB IT NL**

(56) Entgegenhaltungen:
EP - A1 - 0 000 483
EP - A2 - 0 001 473
EP - A1 - 0 002 925
DE - A1 - 2 546 251
DE - A1 - 2 637 886
DE - A1 - 2 732 846

HOUBEN-WEYL, Methoden der organischen Chemie, 4. Auflage, Band IV, Teil 2, 1955 GEORG THIEME VERLAG, Stuttgart, Seiten 518 bis 519

Die Akte enthält technische Angaben die nach dem Eingang der Anmeldung eingereicht wurden und die nicht in dieser Patentschrift enthalten sind.

(73) Patentinhaber: **CIBA-GEIGY AG**
**Patentabteilung Postfach**
**CH-4002 Basel (CH)**

(72) Erfinder: **Brunner, Hans-Georg, Dr.**
**Brunnmattstrasse 6**
**CH-4106 Therwil (CH)**
Erfinder: **Moser, Hans, Dr.**
**Blumenrain 3**
**CH-4465 Magden (CH)**
Erfinder: **Böhner, Beat, Dr.**
**Hügelweg 3**
**CH-4102 Binningen (CH)**
Erfinder: **Föry, Werner, Dr.**
**Benkenstrasse 65**
**CH-4054 Basel (CH)**

(74) Vertreter: **Zumstein sen., Fritz, Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 006 608 B1

Herbizid wirksame, optisch aktive R(+)-Dichlorpyridyloxy-$\alpha$-phenoxy-propionsäure-propargylester, Verfahren zu ihrer Herstellung und deren Verwendung in Mitteln zur Unkrautbekämpfung

Viele aus der Literatur bekannt gewordene herbizid wirksame Derivate von Halogenpyridyloxy-$\alpha$-phenoxy-propionsäuren liegen bei der Verwendung als Wirkstoffe in Form von Racematen vor und sind als solche beschrieben worden z.B. in den DOS 2 546 251, 2 732 846 und in der Japanischen Patentpublikation 1 142 537.

Da solche Verbindungen ein asymmetrisches Kohlenstoffatom besitzen, müssen sie auch als zwei verschiedene optisch aktive Formen mit R- und S-Konfiguration existieren, von denen eine Form häufig biologisch wirksamer ist als das Racemat oder das andere optisch aktive Enantiomere.

In der EP—A 2925 sind einige optisch aktive herbizid wirksame D(+)-Formen von Derivaten der 4-(3',5'-Dichlorpyridyl-(2')-oxy-$\alpha$-phenoxy)-propionsäure als wirkungsmässig den bekannten Racematen überlegen beschrieben worden, sowie Verfahren zur Herstellung dieser D(+)-Enantiomeren.

Die EP—A 2925 beschreibt aber keine D(+)-Formen von Alkinylestern der oben genannten Säure. Die Racemate von Alkinylestern der 4-[3',5'-Dichlorpyridyl-(2')-oxy-)-$\alpha$-phenoxy]-propion- und propionthiol-säure sind aus der Literatur ebenfalls noch nicht vorbekannt. Wohl umfasst die DOS 2 732 846 in ihrem weitestern Umfang Racemate von Alkinylestern konstitutionell nah verwandter 3,5,6-*Tri*halogenpyridyl-(-2)-oxy-$\alpha$-phenoxy-propionsäuren, nennt aber keine Beispiele solcher im Pyridylrest trihalogenierter Propionsäurealkinylester. Von den $\alpha$-[4-(3',5'-Dichlorpyridyl-(2')-oxy)-$\alpha$-phenoxy]-propion- und propionthiol-säurepropargylestern ist weder das Racemat vorbeschrieben noch ein optisch aktives Enantiomer aus der Literatur bekannt geworden.

Die vorliegende Erfindung betrifft nun ein Verfahren zur Herstellung der herbizid wirksamen optischen R(+)-Isomeren der Propargylester der 4-(3',5'-Dichlorpyridyl-(2')-oxy)-$\alpha$-phenoxypropionsäure und der entsprechenden Propionthiolsäure, die beiden neuen optisch aktiven R(+)-Isomeren dieser beiden Propargylester selbst, ferner herbizide Mittel, die diese R(+)-Isomeren als herbizide Wirkstoffe enthalten, sowie die Verwendung dieser optischen R(+)-Enantiomeren und sie enthaltender Mittel zur Unkrautbekämpfung.

Die unter vorliegende Erfindung fallenden optisch aktiven R(+)-isomeren Ester besitzen die Formel I

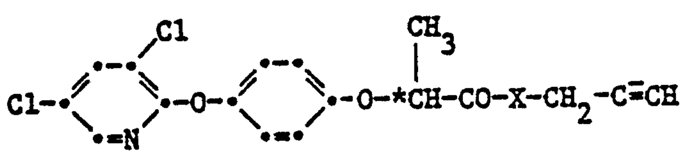

(I),

R(+)-Form

worin X Sauerstoff oder Schwefel darstellt.

Zur Herstellung der beiden genannten Diastereoisomeren der Formel I mit R-Konfiguration kommen zwei an sich bekannte Methoden in Betracht:

1). *Racematspaltung* durch Umsetzung einer racemischen Säure der Formel

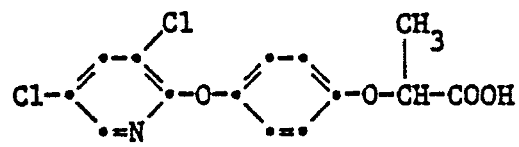

RS (±)

in einem geeigneten Lösungsmittel mit einem optisch aktiven Amin, fraktioniertes Aus- und Umkristallisieren mindestens eines der gebildeten optisch aktiven Salze bis zur Erreichung eines konstanten Schmelzpunkts und konstanter optischer Drehung, gegebenenfalls Freisetzung der entsprechenden optisch aktiven (+) oder (—)-drehenden Säure mit R-Konfiguration aus dem so gewonnenen Salz und Ueberführung dieser Säure in den entsprechenden Propargylester der Formel I, z.B. durch Veresterung mit Propargylalkohol oder durch Umesterung eines zuvor hergestellten anderen Esters.

Dieses Racematspaltungsverfahren ist generell beispielsweise in "Houben-Weyl", Methoden der organischen Chemie *4*.2, S. 518 (1955) beschrieben und jedem Fachmann geläufig.

Als optisch aktive Amine kommen die üblichen in Frage, wie Pflanzenalkaloide und insbesondere als stärkere Basen einfacher gebaute Amine wie (—)-Menthylamin oder das synthetisch leicht zugängliche (+)-bzw. (—)-$\alpha$-Phenyläthylamin, ferner 2-Aminobutan und Benzedrin.

Geeignete Lösungsmittel für die Racematspaltung sind Wasser, Alkohole, Ketone wie Aceton, Acetonitril, Chloroform, Essigester, Benzol etc.

Anstatt Säure und Base wie üblich im Molverhältnis 1:1 anzuwenden, kann man zunächst nur 1/2 Mol Base anwenden, und die restliche Base erst nach Kristallisation des ersten diastereomeren Salzes zusetzen, um die Abscheidung des anderen Salzes zu erreichen.

Die Gewinnung der optisch aktiven Säuren aus den diastereomeren Salzen ist einfach, da die Salze durch Alkalilauge, Ammoniak bzw. Sodalösung oder durch Mineralsäure leicht zerlegbar sind.

2). *Asymmetrische Synthese*

Am besten eignet sich dafür die mit Walden'scher Umkehrung verlaufende stereospezifische Umsetzung des Pyridyloxy-phenols der Formel II

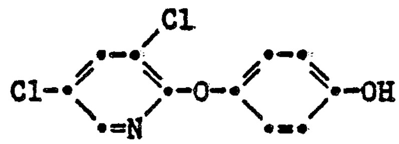

(II)

in Gegenwart einer Base, mit dem S-Isomeren eines Milchsäurederivates der Formel III

$$Y—{}^{*}\underset{\phantom{X}}{\overset{CH_3}{\overset{|}{C}}}H—A' \qquad (III),$$

worin A' eine der Gruppen

$$—CO—OR_1 \text{ oder } —CN,$$

$R_1$ Wasserstoff oder einen niederen Alkylrest und Y eine nucleofuge Gruppe wie Halogen, Alkyl- oder Arylsulfonyloxy —O—$SO_2$—Q, worin Q ein Kohlenwasserstoffrest ist, oder

$$—O—\underset{OH}{\underset{|}{CH}}—C(Hal)_3$$

bedeutet. Bevorzugte nucleofuge Gruppen Y sind Methansulfonyloxy und para-Toluolsulfonyloxy (Tosyloxy).

Solche Umsetzungen zwischen Phenolen und optisch aktiven Milchsäurederivaten mit nucleofugen Gruppen, welche unter Walden'scher Inversion durch den deprotonierten Phenolrest ersetzt werden, sind aus der Literatur bekannt, z.B.: J. Chem. Soc. *127*, 399 (1925), Org. Synth. Coll. Vol. 3, 140 (1955), J. Agric. Food Chem. *23*, 1008 (1975) und DOS 2 734 667.

Nach dieser asymmetrischen Synthese kann das erhaltene optisch aktive Säurederivat, z.B. Ester, Nitril, zur freien Säure verseift und in den entsprechenden Propargylester dieser Säure oder der ihr entsprechenden Thiolsäure übergeführt werden.

Als Basen, welche bei der Umsetzung zwischen Phenol der Formel II und Milchsäurederivat III vorhanden sein müssen, seien Amine, wie Triäthylamin sowie Alkalimetall-Carbonate und Bicarbonate besonders genannt.

Zur Herstellung der optisch aktiven Milchsäurederivate der Formel III mit S-Konfiguration verfährt man entsprechend den Angaben in der obenerwähnten Literatur, z.B. DOS 2 734 667, indem man ein Milchsäurederivat der Formel

$$HO—{}^{*}\underset{CH_3}{\underset{|}{\overset{A'}{\overset{|}{C}}}}—H \qquad (IV)$$

das am mit * markierten C-Atom die S-Konfiguration aufweist, mit einer Verbindung des Typs Hal-$SO_2$—Q, Hal-CO—Q, —OC(OQ)$_2$ oder H—CO—C(Hal)$_3$ umsetzt, wobei Q einen Kohlenwasserstoffrest bedeutet, also mit einem solchen Säurehalogenid, Säureanhydrid oder halogenierten Aldehyd umsetzt. Bevorzugt sind dabei Alkylsulfonylhalogenide, z.B. Methansulfonylchlorid und Arylsulfonylhalogenide wie para-Toluolsulfonylchlorid. Bei diesen Umsetzungen setzt man vorzugsweise organische Basen, wie Pyridin, Triäthylamin zu und arbeitet bei Temperaturen zwischen −5°C und +80°C.

Bei der erfindungsgemässen Umsetzung zwischen dem Phenol der Formel II und beispielsweise dem S-isomeren Milchsäurederivat der Formel III verlässt die Gruppe Y das Molekül und wird durch den deprotonierten Rest des Phenols ersetzt, aber durch Walden'sche Umkehrung an der sterisch inversen Stelle, so dass das Endprodukt die R-Konfiguration aufweist.

Zwar wird hierbei nicht ausschliesslich das gesuchte (inverse) optisch aktive Isomer erhalten, doch liegen die Ausbeuten meist um oder über 80%.

Wie bei allen stereospezifischen Verfahren müssen auch hier die besten Reaktionsbedingungen, Lösungsmittel, Basen etc. für jeden Einzelfall sorgfältig ermittelt und ausgewählt werden, um die optische Integrität des gewünschten Endproduktes möglichst sicher zu stellen. Man kann Lösungsmittel verwenden, wie höhere Aether und Tetrahydrofuran, aber auch Toluol oder Dimethylsulfoxid.

Besonders leicht zugänglich sind Milchsäurederivate der Formel IV, worin A' die Gruppe —$COOR_1$ ist, worin $R_1$ Wasserstoff (Milchsäure) oder ein niederer Alkylrest ist (Milchsäurealkylester). Solche gut zugänglichen Materialien sind beispielsweise S-(+)-Milchsäure, S-(—)-Aethyl-lactat.

Die Endprodukte [R(+)-Enantiomere] der Formel I sind selektive Herbizide, welche sich besonders zur selektiven Bekämpfung schwerbekämpfbaren monocotyler Umgräser in pre- und insbesondere postemergenter Anwendung in Kulturpflanzenbeständen, wie z.B. in Soja, Baumwolle Zuckerrüben etc. eignen. Sie lassen sich auch vorzüglich in Getreidearten wie z.B. Weizen selektiv einsetzen.

Die neuen optisch aktiven Isomeren mit R-Konfiguration, d.h. die herbizid wirksameren Isomeren werden wie racemische Wirkstoffe ähnlicher Konfiguration als herbizide Mittel in bekannten Formulierungsarten angewendet.

Die in dieser Beschreibung zur Bezeichnung der absoluten Konfiguration verwendete Bezeichnung *R* (rectum) und *S* (sinister) richtet sich nach dem Artikel in "Experientia", Band 12, Seiten 81—94 (1956).

Die nachfolgenden Beispiele erläutern die oben beschriebenen Verfahren zur Herstellung der neuen stereoisomeren Propargylester mit R-Konfiguration gemäss Formel I.

### Beispiel 1

a) R(+)-α-[4-(3',5'-Dichlorpyridyl-2'-oxy)-phenoxy]-propionsäure. ($R^{\oplus}$-Säure).

Zu einer Lösung von 200 g racemischer (±)-α-[4-(3',5'-Dichlorpyridyl-2'-oxy)-phenoxy]-propionsäure (DOS 2 546 251) in 500 ml Acetonitril wurden bei 50°C 76,5 g (—)-α-Phenyläthylamin getropft, und das Salz bei Raumtemperatur auskristallisiert. Das Salz wurde aus Acetonitril/Chloroform zu konstantem Schmelzpunkt und konstanter Drehung kristallisiert (Smp.: 165—167°C, $[\alpha]_D^{25} = +23°$ (C = 4%, $CHCl_3$).

Das Salz wurde in 400 ml Essigester und 200 ml 1n-HCl 5 -Minuten gerührt. Die organische Phase wurde abgetrennt, neutral gewaschen, getrocknet und eingeengt. Durch Kristallisieren aus Essigester/Hexan wurde die R(+)-Säure gewonnen (39,9 g, Smp.: 128—129°C, $[\alpha]_D^{25} = +23°$ (C = 3%, $CHCl_3$).

b) Die Ueberführung dieser Säure in den entsprechenden R(+)-Propargylester ist in Beispiel 2d beschrieben.

### Beispiel 2

a) S(—)-α-Tosyloxymilchsäureäthylester.

Zu einer Aufschlämmung von 1 Mol Tosylchlorid (p-Toluolsulfonsäurechlorid) in 1,1 Mol S(—)-Milchsäureäthylester wird bei einer Temperatur von unter 10°C 1 Mol Triäthylamin langsam zugetropft und das Reaktionsgemisch während 15 Stunden bei Raumtemperatur gerührt. Dann verdünnt man mit Aether, filtriert die Salze ab, wäscht die organische Phase, trocknet und engt sie ein. Destillieren am Hochvakuum ergab 215 g S(—)-α-Tosyloxy-milchsäureäthylester vom Kp. 137—139°/0,03 mb; $[\alpha]_D^{25} = -53°$.

b) Aethylester der R(+)-Säure

14,5 g 4-(3',5'-Dichlorpyridyl-2'-oxy)-phenol, 14,8 g S(—)-α-Tosyloxymilchsäureäthylester und 5 g $K_2CO_3$ werden in zwei Ansätzen einerseits mit 70 ml Dimethylsulfoxid auf 60°C erwärmt und andererseits in 100 ml Toluol am Rückfluss gekocht. Die Reaktionslösungen werden mit Aether verdünnt, gewaschen, getrocknet und eingeengt. Nach Chromatographie erhält man 16,5 g R(+)-α-[4(3,5'-Dichlorpyridyl-2'-oxy)-phenoxy]-propionsäureäthylester als Oel mit $[\alpha]_D^{25} = +34°$ (C = 4%, $CHCl_3$).

c) freie R(+)-Säure

14,5 g des so erhaltenen R(+)-Säureäthylesters werden in 50 ml Methanol nach Zugabe von 50 ml 2nKOH 15 Stunden bei Raumtemperatur gerührt. Das Methanol wird dann abgedampft und der Rückstand mit konzentrierter Salzsäure sauer gestellt und mit Essigester extrahiert. Die organische Phase wird dann gewaschen, getrocknet und eingeengt, und die R(+)-Säure aus Essigester/Hexan kristallisiert.

Man erhält so 11,9 g der R-(+)-Säure, welche mit der gemäss Beispiel 1 erhaltenen Säure identisch ist und bei 120—127°C schmilzt; $[\alpha]_D^{25} = +20°C$ (C = 3%, $CHCl_3$).

d) 6 g der auf diese Weise oder gemäss Beispiel 1a erhaltenen R(+)Säure, 5 ml Propargylalkohol und 100 mg p-Toluolsulfonsäure werden während 5 Stunden in 500 ml Toluol am Wasserabscheider gekocht. Die Reaktionslösung wird dann mit einer wässerigen Natriumbicarbonatlösung und mit Wasser gewaschen, dann getrocknet und eingeengt. Es wurden so 5,8 eines Oeles erhalten, das beim Stehenlassen kristallisierte: Smp.: 66—68°C; $[\alpha]_D^{25} = + 36° \pm 1°$ (3%, $CHCl_3$).

Es handelt sich um die R(+)-Form des Propargylesters der Formel

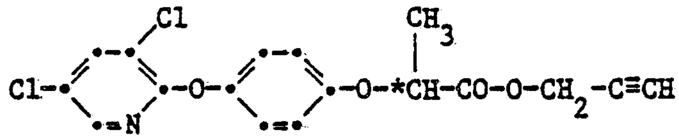

Beispiel 3

a) 10 g der R(+)-Säure, hergestellt nach Beispiel 1a oder 2(a-c) werden in 100 ml Toluol suspendiert und nach Zutropfen von 8 ml Oxalylchlorid 3 Stunden bei Raumtemperatur gerührt. Das dabei entstehende Säurechlorid wird durch Einengen am Rotavap gewonnen.

b) Eine Lösung von 8,9 g KOH, 5 ml Wasser und 75 ml Dimethoxyäthan wird bei 15°C mit $H_2S$ gesättigt. Zu dieser Lösung lässt man unter Eiskühlung das unter a) erhaltene rohe Säurechlorid in 10 ml Dimethoxyäthan zutropfen, worauf noch 30 Minuten bei Raumtemperatur gerührt wird. Die Reaktionslösung wird dann auf Eis gegossen, mit konzentrierter Salzsäure angesäuert auf pH~1 und das entstandene Produkt mit Methylenchlorid extrahiert. Säulenchromatographie an $SiO_2$ mit Methylenchlorid ergab 10,3 g *Thiolsäure* vom Schmelzpunkt 70—75°C; $[\alpha]_D = +34° \pm 1°$(3%, $CHCl_3$).

c) Zu 10 g dieser Thiolsäure und 4,5 g Kaliumcarbonat in 50 ml Methyläthylketon werden 3,9 g Propargylbromid gegeben, worauf ein schwach exotherme Reaktion einsetzt. Nach 30-minütigem Rühren bei Raumtemperatur wird die Reaktionslösung filtriert und eingeengt. Filtration an wenig $SiO_2$ und Kristallisation aus Aether/Petroläther ergaben 7,1 g des R(+)-Thiopropargylesters der Formel

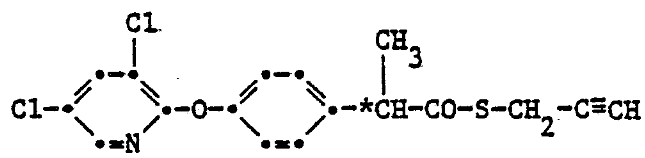

Schmelzpunkt: 75—80°C; $[\alpha]_D^{25} = +40° \pm 1°$ (3%, $CHCl_3$).

Die enantiomere R(+)-Form ist der anderen stereoisomeren S(—)-Form und vermutlich auch dem noch nicht vorbeschriebenen Racemat in der herbiziden Wirksamkeit überlegen. Die neuen R(+)-Propargylester und die sie enthaltenden herbiziden Mittel sind insbesondere brauchbar zu selektiven Bekämpfung schwer bekämpfbarer Unkräuter wie Avena fatua in Kulturpflanzenbeständen, sowohl in dicotylen als auch in monocotylen Kulturpflanzen wie Weizen etc.

Die aktivere stereoisomere Form mit R-Konfiguration zeichnet sich gegenüber gewissen bekannten Racematen aus der Reihe der gesättigten Ester und Thiolester durch bessere Aktivität gegen Ungräsern aus.

Die Erfindung betrifft auch herbizide Mittel, welche das aktivere R(+)-Enantiomere der Formel I enthalten, sowie Verfahren zur pre- oder post-emergenten Unkrautbekämpfung.

Die erfindungsgemässen Mittel können in den üblichen Formulierungen vorliegen und werden in an sich bekannter Weise hergestellt durch Vermischen und Vermahlen von optisch aktiven Wirkstoffen der Formel I mit geeigneten Trägerstoffen, gegebenenfalls unter Zusatz von Dispersions- und Lösungsmitteln. In dieser Weise werden nach bekannten Techniken Stäubemittel, Granulate, Spritzpulver, Pasten, Emulsionen, emulgierbare Konzentrate oder Lösungen hergestellt.

Die Aufwandmengen bei der Bekämpfung von Unkräutern betragen in der Regel 0,1 bis 10 kg Wirkstoff pro ha, vorzugsweise 0,25 bis 5 kg/ha.

Zum Nachweis der Brauchbarkeit dienen die üblichen allgemein bekannten Teste für pre- und post-emergente Anwendung.

**Patentansprüche für die Vertragsstaaten: BE, CH, DE, FR, GB, IT, NL**

1. Neue optisch aktive R(+)-isomere Propargylester der $\alpha$-4-[(3',5'-Dichlorpyridyl-(2')-oxy)-phenoxy]-propion- und propionthiol-säure der Formel I

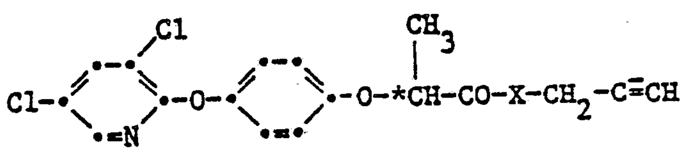 (I),

R(+)-Form

worin X Sauerstoff oder Schwefel darstellt.

2. R(+)-$\alpha$-[4-(3',5'-Dichlorpyridyl-2'-oxy)-phenoxy]-propionsäurepropargylester.

3. Verfahren zur Herstellung der optische aktiven R-Form des Propargylesters des Patentanspruchs 2, dadurch gekennzeichnet, dass man die racemische ($\pm$)-Dichlorpyridyloxy-$\alpha$-phenoxypropionsäure der Formel

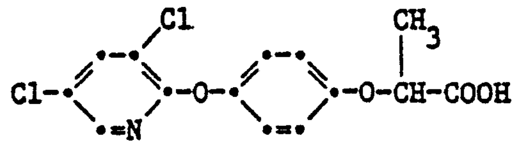

in einem Lösungsmittel in an sich bekannter Weise mit einem optisch aktiven Amin umsetzt, von den beiden gebildeten optisch aktiven Salzen durch fraktionierte Aus- und Umkristallisation bis zur Erreichung eines konstanten Schmelzpunktes das R-Salz abtrennt und aus diesem Salz die entsprechende R-Säure freisetzt und diese Säure mit Propargylalkohol verestert. .

4. Verfahren gemäss Patentanspruch 3, dadurch gekennzeichnet, dass man als optisch aktives Amin das (—)-$\alpha$-Phenyläthylamin verwendet.

5. Verfahren zur Herstellung der enantiomeren R-Form der Propargylester der Formel I des Patentanspruchs 1, dadurch gekennzeichnet, dass man das Dichlor-Pyridyloxy-phenol der Formel II

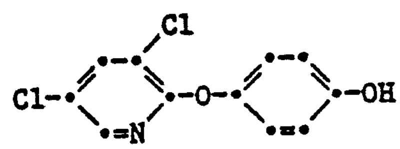

(II)

in Gegenwart einer Base mit einem optisch aktiven Milchsäurederivat der Formel III

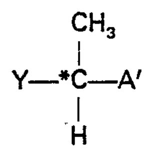

(III)

welches am *C die S-Konfiguration aufweist und worin A' eine der Gruppen —$COOR_1$ oder —CN und $R_1$ Wasserstoff oder einen niederen Alkylrest darstellt, und Y eine nucleofuge Gruppe bedeutet, umsetzt, wobei diese nucleofuge Gruppe Y in Form einer Verbindung HY austritt und durch den deprotonierten Rest des genannten Phenols II an der optisch inversen Stelle des *C Atoms ersetzt wird, und dass man das so erhaltene optisch aktive Derivat in die freie R(+)-Propionsäure bzw. R(+)-Propionthiolsäure überführt und man aus dieser Säure dann den Propargylester herstellt.

6. Verfahren gemäss Patentanspruch 5, dadurch gekennzeichnet, dass im Milchsäurederivat der Formel III die zu ersetzende Gruppe Y der Tosyloxyrest ist.

7. Herbizides Mittel, dadurch gekennzeichnet, dass es als wirksame Komponente das R(+)-Enantiomere eines Propargylesters der Formel I des Patentanspruchs 1 enthält.

8. Verwendung des R(+)-Enantiomeren eines Propargylesters der Formel I des Patentanspruchs 1 als Wirkstoff in herbiziden Mitteln zur Unkrautbekämpfung.

**Patentansprüche für den Vertragsstaat: AT**

1. Verfahren zur Herstellung neuer optisch aktiver R(+)-isomerer Propargylester der $\alpha$-4-[(3',5'-Dichlorpyridyl-(2')-oxy)-phenoxy]-propion- und propionthiol-säure der Formel I

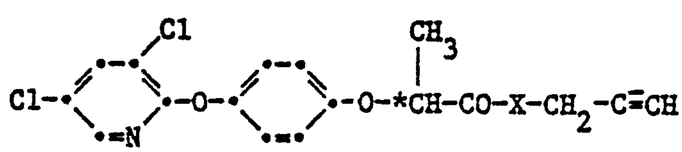

(I)

R(+)-Form

worin X Sauerstoff oder Schwefel darstellt, dadurch gekennzeichnet, daß man das Dichlor-Pyridyloxy-phenol der Formel II

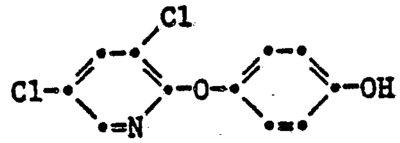

(II)

in Gegenwart einer Base mit einem optisch aktiven Milchsäurederivat der Formel III

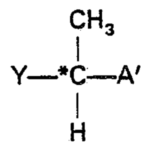

(III)

welches am *C die S-Konfiguration aufweist und worin A' eine der Gruppen —$COOR_1$ oder —CN und $R_1$ Wasserstoff oder einen niederen Alkylrest darstellt, und Y eine nucleofuge Gruppe bedeutet, umsetzt, wobei diese nucleofuge Gruppe Y in Form einer Verbindung HY austritt und durch den deprotonierten Rest des genannten Phenols II an der optisch inversen Stelle des *C Atoms ersetzt wird, und dass man das so erhaltene optisch aktive Derivat in die freie R(+)-Propionsäure bzw. R(+)-Propionthiolsäure überführt und man aus dieser Säure dann den Propargylester herstellt.

2. Verfahren gemäß Patentanspruch 1, dadurch gekennzeichnet, daß im Milchsäurederivat der Formel III die zu ersetzende Gruppe Y der Tosyloxyrest ist.

3. Verfahren gemäß Anspruch 1 oder 2, dadurch gekennzeichnet, daß man R(+)-α-[4-(3',5'-Dichlorpyridyl-2'-oxy)-phenoxy]-propionsäure-propargylester herstellt.

4. Verfahren zur Herstellung der optisch aktiven R-Form des Propargylesters des Patentanspruchs 3, dadurch gekennzeichnet, dass man die racemische (±)-Dichlorpyridyloxy-α-phenoxy-propionsäure der Formel

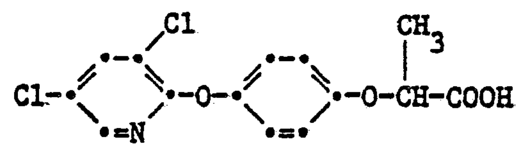

in einem Lösungsmittel in an sich bekannter Weise mit einem optisch aktiven Amin umsetzt, von den beiden gebildeten optisch aktiven Salzen durch fraktionierte Aus- und Umkristallisation bis zur Erreichung eines konstanten Schmelzpunktes das R-Salz abtrennt und aus diesem Salz die entsprechende R-Säure freisetzt und diese Säure mit Propargylalkohol verestert.

5. Verfahren gemäß Patentanspruch 4, dadurch gekennzeichnet, daß man als optisch aktives Amin das (—)-α-Phenyläthylamin verwendet.

6. Herbizides Mittel, dadurch gekennzeichnet, daß es als wirksame Komponente das R(+)-Enantiomere eines Propargylesters der Formel I des Patentanspruchs 1 enthält.

7. Verwendung des R(+)-Enantiomeren eines Propargylesters der Formel I des Patentanspruchs 1 als Wirkstoff in herbiziden Mitteln zur Unkrautbekämpfung.

**Claims for the Designated States: BE, CH, DE, FR, GB, IT, NL**

1. Novel optically active R(+)isomeric propargyl esters of α-4-[3',5'-dichloropyridyl-(2')-oxy)phenoxy]propionic and thiopropionic acid of the formula I

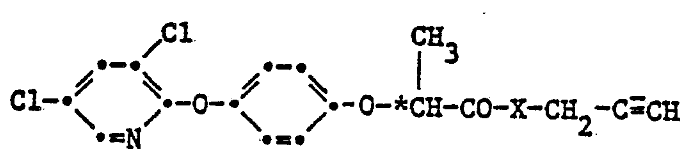

(I)

R(+)-Form

wherein X is oxygen or sulfur.

2. R(+)-α-[4-(3',5'-Dichloropyridyl-2'-oxy)phenoxy]propionic acid propargyl ester.

3. A process for the production of the optically active R-form of the propargyl ester of claim 2,

which process comprises reacting the racemic (±)-dichloropyridyloxy-$\alpha$-phenoxypropionic acid of the formula

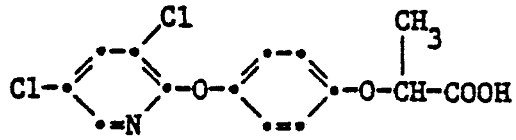

in a solvent, in a manner known per se, with an optically active amine, separating the R-salt from the two resultant optically active salts by fractional crystallisation and recrystallisation until the melting point is constant, liberating the corresponding R-acid from said R-salt, and esterifying said acid with propargyl alcohol.

4. A process according to claim 3, wherein (—)-$\alpha$-phenylethylamine is used as optically active amine.

5. A process for the production of the enantiomeric R-form of the propargyl esters of the formula I according to claim 1, which process comprises reacting the dichloropyridyloxy phenol of the formula II

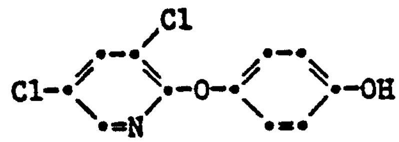

(II)

in the presence of a base, with an optically active lactic acid derivative of the formula III

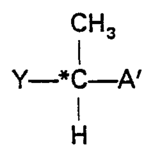

(III)

which has the S-configuration at the *C atom and wherein A' is one of the groups —$COOR_1$ or —CN and $R_1$ is hydrogen or a lower alkyl radical, and Y is a leaving group which leaves the molecule in the form of a compound HY and is replaced by the deprotonated radical of the phenol of the formula II at the optically inverse position of the *C atom, and converting the resultant optically active derivative into the free R(+)-propionic acid or R(+)-thiopropionic acid, and then preparing the propargyl ester from said acid.

6. A process according to claim 5, wherein the leaving group Y in the lactic acid derivative of the formula III is the tosyloxy group.

7. A herbicidal composition which contains, as active component, the R(+)-enantiomer of a propargyl ester of the formula I according to claim 1.

8. Use of the R(+)-enantiomer of a propargyl ester of the formula I according to claim 1 as active ingredient in herbicidal compositions for controlling weeds.

**Claims for the Contracting State: AT**

1. A process for the production of novel optically active R(+)-isomeric propargyl esters of $\alpha$-4-[3',5'-dichloropyridyl-(2')-oxy)phenoxy]propionic and thiopropionic acid of the formula I

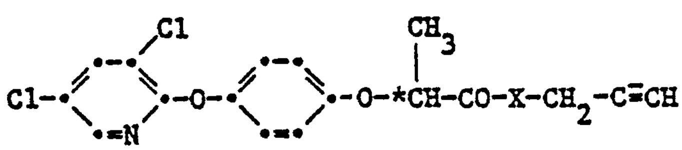

(I)

R(+)-Form

wherein X is oxygen or sulfur, which process comprises reacting the dichloropyridyloxy phenol of the formula II

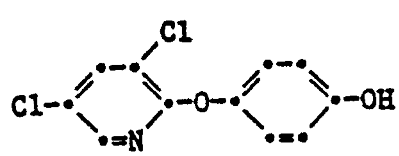

(II)

in the presence of a base, with an optically active lactic acid derivative of the formula III

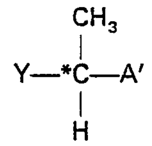

(III)

which has the S-configuration at the *C atom and wherein A' is one of the groups —$COOR_1$ or —CN and $R_1$ is hydrogen or a lower alkyl radical, and Y is a leaving group which leaves the molecule in the form of a compound HY and is replaced by the deprotonated radical of the phenol of the formula II at the optically inverse position of the *C atom, and converting the resultant optically active derivative into the free R(+)-propionic acid or R(+)-thiopropionic acid, and then preparing the propargyl ester from said acid.

2. A process according to claim 1, wherein the leaving group Y in the lactic acid derivative of the formula III is the tosyloxy group.

3. A process according to claim 1 or 2 for the production of R(+)-$\alpha$-[4-(3',5'-dichloropyridyl-2'-oxy)phenoxy]propionic acid propargyl ester.

4. A process for the production of the optically active R-form of the propargyl ester of claim 3, which process comprises reacting the racemic (±)-dichloropyridyloxy-$\alpha$-phenoxypropionic acid of the formula

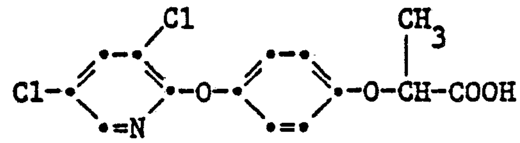

in a solvent, in a manner known per se, with an optically active amine, separating the R-salt from the two resultant optically active salts by fractional crystallisation and recrystallisation until the melting point is constant, liberating the corresponding R-acid from said R-salt, and esterifying said acid with propargyl alcohol.

5. A process according to claim 4, wherein (—)-$\alpha$-phenethylamine is used as optically active amine.

6. A herbicidal composition which contains, as active component, the R(+)-enantiomer of a propargyl ester of the formula I according to claim 1.

7. Use of the R(+)-enantiomer of a propargyl ester of the formula I according to claim 1 as active ingredient in herbicidal compositions for controlling weeds.

**Revendications pour les états contractants: BE, CH, DE, FR, GB, IT, NL**

1. Nouveaux esters propargyliques isomères optiques R(+) des acides alpha-4-[(3',5'-dichloro-pyridyl-(2')-oxy)-phénoxy]-propionique et -thio-propionique de formule I:

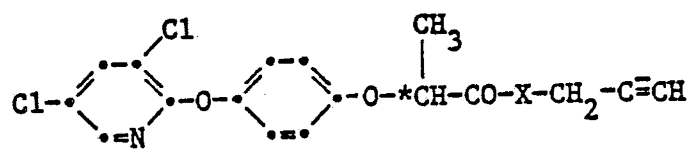

(I)

Forme R(+)

dans laquelle X représente l'oxygène ou le soufre.

2. Le R(+)-alpha-[4-(3',5'-dichloropyridyl-2'-oxy)-phénoxy]-propionate de propargyle.

3. Procédé de préparation de la forme optique R de l'ester propargylique de la revendication 2, caractérisé en ce que l'on fait réagir l'acide (±)-dichloropyridyloxy-alpha-phénoxy-propionique racémique de formule:

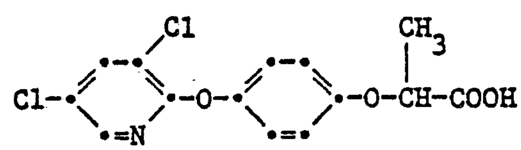

dans un solvant, de manière connue en soi, avec une amine énantiomère, on sépare des deux sels optiques formés, par cristallisation et recristallisation fractionnées jusqu'à point de fusion constant, le

sel R, on libère de ce sel l'acide R correspondant et on estérifie cet acide par l'alcool propargylique.

4. Procédé selon la revendication 3, caractérisé en ce que l'on utilise en tant qu'amine énantiomère la (—)-alpha-phényléthylamine.

5. Procédé de préparation de la forme énantiomère R de l'ester propargylique de formule I de la revendication 1, caractérisé en ce que l'on fait réagir le dichloropyridyloxy-phénol de formule II:

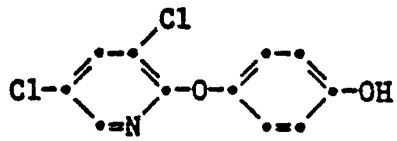

(II)

en présence d'une base avec un dérivé énantiomère de l'acide lactique de formule III:

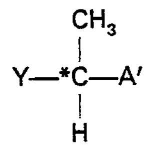

(III)

présentant la configuration S sur l'atome de carbone *C et dans laquelle A' représente l'un des groupes —$COOR_1$ ou —CN, $R_1$ représentant l'hydrogène ou un groupe alkyle inférieur, et Y représente un groupe nucléofuge, ce groupe nucléofuge Y étant éliminé sous la forme d'un composé HY et remplacé par le reste déprotonisé du phénol II mentionné à l'endroit optiquement inverse de l'atome *C, on convertit le dérivé énantiomère ainsi obtenu en l'acide R(+)-propionique ou R(+)-thiol-propionique libre et on prépare l'ester propargylique à partir de cet acide.

6. Procédé selon la revendication 5, caractérisé en ce que le groupe Y à remplacer dans le dérivé d'acide lactique de formule III est le reste tosyloxy.

7. Produit herbicide caractérisé en ce qu'il contient en tant que composant actif l'énantiomère R(+) d'un ester propargylique de formule I selon la revendication 1.

8. Utilisation de l'énantiomère R(+) d'un ester propargylique de formule I selon la revendication 1 en tant que substance active dans des produits herbicides pour la lutte contre les mauvaises herbes.

**Revendications pour l'état contractant: AT**

1. Procédé de préparation de nouveaux esters propargyliques isomères optiques R(+) de l'acide alpha-4-[(3',5'-dichloropyridyl-(2')-oxy)-phenoxy]-propionique et -thio-propionique de formule I:

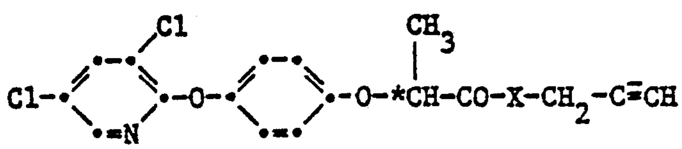

(I)

Forme R(+)

dans laquelle X représente l'oxygène ou le soufre, caractérisé en ce que l'on fait réagir le dichloropyridyloxyphénol de formule II:

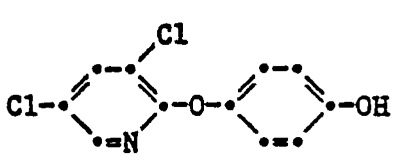

(II)

en présence d'un base, avec un dérivé énantiomère de l'acide lactique de formule III:

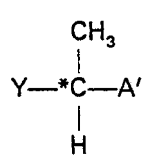

(III)

qui présente la configuration S sur l'atome de carbone *C et dans laquelle A' représente l'un des groupes —$COOR_1$ ou —CN, $R_1$ représentant l'hydrogène ou un groupe alkyle inférieur, et Y représente un groupe nucléofuge, ce groupe nucléofuge Y étant éliminé sous la forme d'un composé HY et

remplacé par le reste déprotonisé du phénol II mentionné à l'endroit optiquement inverse de l'atome *C, on convertit le dérivé optique obtenu en l'acide R(+)-propionique ou R(+)-thiol-propionique libre et on prépare à partir de cet acide l'ester propargylique.

2. Procédé selon la revendication 1, caractérisé en ce que le groupe Y à remplacer dans le dérivé d'acide lactique de formule III est le reste tosyloxy.

3. Procédé selon la revendication 1 ou 2, caractérisé en ce que l'on prépare le R(+)-alpha-[4-(3',5'-dichloropyridyl-2'-oxy)-phénoxy]-propionate de propargyle.

4. Procédé de préparation de la forme optique R de l'ester propargylique selon la revendication 3, caractérisé en ce que l'on fait réagir l'acide (±)-dichloropyridyloxy-alpha-phénoxy-propionique racémique de formule:

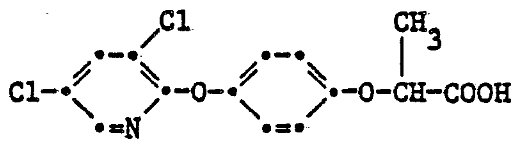

dans un solvant, de manière connue en soi, avec une amine énantiomère, on sépare le sel R des deux sels optiques formés par cristallisation et recristallisation fractionnées jusqu'à point de fusion constant et à partir de ce sel, on libère l'acide R correspondant qu'on estérifie par l'alcool propargylique.

5. Procédé selon la revendication 4, caractérisé en ce que l'on utilise en tant qu'amine énantiomère la (—)-alpha-phényléthylamine.

6. Produit herbicide caractérisé en ce qu'il contient en tant que composant actif l'énantiomère R(+) d'un ester propargylique de formule I selon la revendication 1.

7. Utilisation de l'énantiomère R(+) d'un ester propargylique de formule I selon la revendication 1, en tant que substance active dans des produits herbicides pour combattre les mauvaises herbes.